# EUROPEAN PATENT APPLICATION

(11) **EP 2 008 620 A2**
(43) Date of publication of application: **31.12.2008**
(21) Application number: 08103811.9
(22) Date of filing: 02.05.2008
(51) Int. Cl.: A61F 2/34

(54) **Prosthesis**

(30) Priority: 28.02.2008 GB 0803676; 26.06.2007 EP 07111092
(71) Applicant: Finsbury (Development) Limited, Leatherhead, Surrey KT22 0BA (GB)
(72) Inventor: Taylor, Andrew Clive, Chichester, Surrey PO19 3QQ (GB); Tuke, Michael Anthony, Guildford, Surrey GU1 3TF (GB)
(74) Representative: Smaggasgale, Gillian Helen

(57) **Abstract**

A preassembled acetabular cup prosthesis comprising:
an outer shell; and
a liner located within the shell.

## Description

The present invention relates to a prosthesis. More particularly, it relates to a preassembled acetabular component for a hip prosthesis and a process for the production thereof.

The efficient functioning of the hip joint is extremely important to the well-being and mobility of the human body. Each hip joint is comprised by the upper portion of the femur which terminates in an offset bony neck surmounted by a ball-headed portion which rotates within the acetabulum in the pelvis. Diseases such as rheumatoid- and osteo-arthritis can cause erosion of the cartilage lining of the acetabulum so that the ball of the femur and the hip bone rub together causing pain and further erosion. Bone erosion may cause the bones themselves to attempt to compensate for the erosion which may result in the bone becoming misshapen.

Operations to replace the hip joint with an artificial implant are well-known and widely practiced. Generally, the hip prosthesis will be formed of two components, namely: an acetabular component which lines the acetabulum; and a femoral component which replaces the femoral head. The femoral component may be total femoral head replacement in which case the component includes a head, neck and a stem which in use in inserted into the end of a prepared femur. Alternatively, where appropriate, the femoral head component may be a resurfacing prosthesis which is attached to the head of the femur once it has been suitably machined.

In an operation to insert a prosthetic acetabulum in a patient's pelvis the surgeon first uses a reamer to cut a cavity of appropriate size in the patient's pelvis. An acetabular cup is then inserted into the cavity. By "appropriate size" is meant a size which is selected by the surgeon as being the most appropriate for that particular patient. Normally, it is desirable to retain as much of the original healthy bone surface as possible.

Commercially available acetabular cups are sold in a range of sizes to suit the needs of individual patients. Generally, acetabular cups are available in sizes of from 42 mm to 62 mm diameter with 2 mm increments between neighboring sizes.

There are a number of different types of prosthetic acetabular cups. One type of cup is those made from polyethylene. They are generally cemented into the acetabulum and require only light pressure to seat them in the cement.

One alternative cup type has a polyethylene liner unit for articulation with the femur and a metal shell for insertion into the pelvic cavity. These cups with metal shells may be implanted without cement such that they rely on a jam fit between the metal shell and the patient's acetabulum. However, in some arrangements, screws may be used to secure the cup shell in position in the pelvis before the liner is applied into position. The insertion of the metal shell into the pelvis requires considerable force. As the surgeon applies this force, there is a risk that the metal shell can become damaged or deformed. There is also a possibility that during the application of the force, the shell may be moved so that it is not in the optimum alignment in the acetabulum. Often the metal shells have outer surfaces or coatings which encourage bone to grow into them over time.

With this type of prosthesis, the polyethylene liner unit is snapped or screwed into the metal shell after the metal shell has been seated in the acetabulum. Thus the inner surface of the liner forms the socket part of the joint.

More recently, ceramics have been used to as an alternative to the plastics liner. In this arrangement, the metal shell, which is generally formed from titanium and which is of a similar thickness to the arrangement in which a polyethylene liner is used, is inserted into the acetabulum. The ceramic liner is then inserted into the shell. It can be difficult for the liner to be accurately aligned in the shell. In addition, this insertion of the liner does require the application of a considerable force which is usually applied by the surgeon using a mallet often via an insertion tool. Considerable force is generally required to achieve a successful interface. However, this force can damage the ceramic liner.

In order to get an optimum fit, it is necessary that the forces applied for both the insertion of the metal shell and for the ceramic liner are appropriate but not excessive. One problem however, is that to date there has been no understanding as to what forces are appropriate nor is there a means to ensure that the correct force is applied.

The surgeon is not generally able to apply a controlled amount of force applied. Some surgeons may not apply sufficient force in one hit and it may be necessary for a plurality of hits to be used. These may not all strike at the same angle and may not each apply the same force. Other surgeons may apply a much greater single strike. The force applied by the surgeon on, for example, an insertion tool may vary considerably and can be of the order of about 3 to 5 kN but can also be much higher and may even be of the order of about 35 kN.

Whilst very large forces may only be applied for small moments in time, of the order of a few seconds or less, forces of this magnitude, or a plurality of forces of smaller magnitude may cause the shell to be deformed as it is inserted into the acetabulum. This is a particular risk in those arrangements where the thickness of the shell is only from about 1 mm to about 3 mm thick. If the shell is deformed, it can become difficult or even impossible to insert the liner.

Whichever arrangement of form of cup to liner, the liner may be incorrectly seated in the shell which can lead to various disadvantages. Not only is there a risk that where a portion of the liner stands above the rim of the cup, a point of irritation can be produced but also, there is a risk that material, such as wear debris, may congregate against the raised portion of the liner or against the wall of the cup in the area where the liner sits below the rim. This accumulation of debris may provide a site for post-operative infection. Even if the liner is correctly located and the shell is not deformed during the assembly process, it may become deformed on insertion of the prosthesis into the pelvis such that the shell may become spaced from the liner over at least a portion of the prosthesis.

Even if the surgeon is able to accurately seat the liner in the cup, there is a risk that during assembly debris may be caught between the liner and the cup which may effect the wear properties of the prosthesis. A further problem associated with the presence of debris, which may include fluids such as blood or fat, between the shell and liner is that in use, in vivo, the presence of the debris may cause the shell and liner to move apart.

Without wishing to be bound by any particular theory, it will be understood where the shell and ceramic liner are held together by friction, debris, in particular fatty substances or blood, can interfere with the frictional interface between the outer surface of the liner and the inner surface of the shell such that there is a propensity for the liner to move out of the shell.

A further problem which may be encountered is that while inserting the liner in the shell it may become damaged. This is particularly a problem where the liner is formed from ceramic. If this damage is a chip or crack on the outer surface of the liner, i.e. on the surface adjacent to the surface of the shell, its presence may not be noticed by the surgeon during assembly. However, its existence will be a point of weakness which can result in the prosthesis failing in use.

It is therefore desirable to provide an acetabular component which reduces the risk of liner misplacement and which has enhanced life expectancy arising, in part, through improved resistance to damage caused during impaction into the acetabulum. It is also desirable to provide an acetabular cup prosthesis which can be easily handled and inserted during surgery without damage to the acetabular cup prosthesis and which minimizes the risk of debris being trapped between the cup and the liner.

Thus, according to the present invention, there is provided a preassembled unit acetabular cup prosthesis comprising:
an outer shell; and
a liner located within the shell.

Since the unit is preassembled, the liner can be positioned in the outer shell in a controlled manner preferably at the manufacturing site. This will enable the positioning of the liner in the shell to be carefully controlled and checked. Further, the liner can be located within the outer shell in a sterile environment thereby minimizing the risk of debris being located between the liner and the outer shell of the prosthesis. Further since assembly is not only ex-vivo but generally occurs in an area remote from the patient, there is no risk of blood, fat and the like becoming located between the shell and the liner. This is an important advantage. As described above, the presence of such materials in a prosthesis provides a push-off load which can cause separation of the components when loaded in vivo. Thus the prostheses of the present invention have improved structural integrity in vivo.

The outer shell of the prosthesis is preferably made from metal. Any suitable metal may be used, with titanium being particularly preferred. Cobalt/chromium may also be used. The outer surface of the shell may be configured to promote bone integration. In one arrangement, the outer surface may be coated with a bone growth promoting material such as hydroxyapatite. Any suitable thickness of shell may be used.

In a preferred arrangement, the shell is a titanium shell. In a still preferred arrangement, the titanium shell has a thickness in the region of about 1 mm to about 3 mm.

The liner may be made from any suitable material. It one arrangement it may be made from plastics such as polyurethane.

In an alternative arrangement, it may be made from ceramic material. For the purposes of this application, the term "ceramic" should be construed as meaning not only true ceramic materials but also other materials which display ceramic-like properties. Ceramic-like properties for the purposes of the present invention are those where strength, stiffness and rigidity are similar to those of ceramics.

The ceramic liner may be formed of any material which has acceptable biocompatibility, hardness and wear resistance. Suitable ceramic materials include silicon nitride, doped silicon nitride, an alumina-zirconia ceramic, yttria, stabilized zirconia, ceria, stabilized zirconia, zirconia ceramics, alumina ceramics, oxinium or mixtures thereof. The thickness of the liner is preferably in the region of from about 2 mm to about 5 mm.

In one arrangement, securement means may be formed on the inner surface of the outer shell of the cap, on the liner, or on both such that the liner and the shell are a snap-fit together. In one arrangement some threading may be provided over at least a portion of the mating surfaces so that the liner may be threaded into the shell.

However, in a preferred arrangement, the liner may simply be held in position in the outer cup as a tight fit. In this arrangement it will be the friction between the mating surfaces of the cup and the liner which holds them in position. In this connection, the surface roughness of the inner surface of the shell and that of the outer surface of the liner can be selected to optimise the frictional interplay between the surfaces. In one arrangement the shell may have a surface roughness of its inner surface of from about 1 to about 8 µm, preferably from about 2 to about 5 µm and more preferably from about 2.5 to about 4 µm. The outer surface of the liner may have a surface roughness of from about 0.5 to about 3 µm, more preferably from about 1 to about 2.5 µm. In one alternative arrangements coatings may be applied to one or both of the mating surfaces to provide the desired frictional interface. It will be understood that in arrangements relying on friction, other additional connection means may be present but this is not preferred.

The inner surface of the outer shell and the outer surface of the liner may be configured so that there is a corresponding taper fit arrangement. Any suitable taper arrangement may be used. In one arrangement, the taper of the shell, which will be a female type taper, will be of the order of from about 15° to about 20° with tapers in the region of 17° to 19° being preferred. The taper of the liner, which will be a male type taper, will be of the order of about 15° to about 20° with tapers in the region of 17° to 19° being preferred. It should be understood that the tapers may not be identical. In one arrangement, the male taper of the liner may be broader than that of the female taper in the shell such that as the liner is applied into the shell it causes some adjustment in the shape of the thin walled shell. The tapered arrangement is particularly suitable where the liner is a ceramic liner.

The prosthesis of the present invention may be found by any suitable means. In one arrangement, it may be found by the process described in co-pending application no. 08103809.3 filed on even date and also entitled 'Prosthesis' having attorney docket reference (509506).

Other processes may be used for forming the prosthesis of the present invention. In one arrangement, the metal shell may be of a smaller or similar diameter than the liner. In this arrangement, the shell may be heated and then the ceramic liner may be forced into position. As the shell cools it will hold the ceramic liner tightly in position.

The cup and the liner may be connected together using an adhesive. In one arrangement the adhesive may be an anaerobic adhesive i.e. an adhesive which cures when air is excluded. Thus the adhesive will not set until after the cup and liner have been assembled. In one alternative arrangement the adhesive may be cured by other means including by means of heat, light, moisture or radiation curing. Where light is to be used to cure the adhesive, the shell and/or the lines may be configured to allow light to pass therethrough.

Any suitable adhesive may be used although a generally medical grade adhesive will generally be used. The adhesive may comprise more than one component. These may be premixed before the adhesive is used or they may be mixed in situ. For example, one component of the adhesive may be applied to the inner surface of the shell and the other component of the adhesive may be applied to the outer surface of the liner such that when the shell and liner are assembled, the two components of the adhesive come into mutual contact such that curing can occur.

Examples of suitable adhesives include natural and synthetic adhesives. The adhesive may be thermoset, thermoplastic or elastomeric adhesives. Thermoset adhesives include epoxies, polyesters and phenolic resins. Thermoplastic adhesives include polyamines. Elastomers include rubber, styrene copolymers, and the like. Acrylic adhesives such as cyanoacrylates may offer various advantages. Other suitable adhesives include epoxy resins, polyurethane adhesives, or silicones.

In one arrangement pressure sensitive adhesives may be used. Such pressure sensitive adhesives are well known in the art.

One benefit of the use of an adhesive is that the shell and liner need not necessarily be an engineered fit.

In one arrangement, a material may be included in a space between the liner and the shell located at the base of the prosthesis. Suitable materials include acoustic damping materials such as those described in copending application GB 0803676.6 which is incorporated herein by reference.

Since the prosthesis of the present invention is preassembled, the orientation/alignment between the shell and the liner can be optimised and controlled such that an improved prosthesis is provided to the patient. In addition, each of the problems identified above is overcome by this arrangement.

The preassembled prosthesis of the present invention may be provided with an impaction cap. The impaction cap may be pre-assembled with the prosthesis as described in co-pending application EP 07111092.8 which is incorporated herein by reference. In one arrangement, the impaction cap is configured such that the force applied to impact the prosthesis into the acetabular is directed via the ceramic liner so that any shock passing through the metal shell is minimised.

## Claims

1. A preassembled acetabular cup prosthesis comprising:
an outer shell; and
a liner located within the shell.

2. A preassembled acetabular cup prosthesis according to Claim 1 wherein the outer shell of the prosthesis is made from metal.

3. A preassembled acetabular cup prosthesis according to Claim 1 or 2 wherein the liner is made from plastics.

4. A preassembled acetabular cup prosthesis according to any one of Claims 1 to 3 wherein the liner is made from ceramic.

5. A preassembled acetabular cup prosthesis according to Claim 4 wherein the ceramic liner is formed from silicon nitride, doped silicon nitride, an alumina-zirconia ceramic, yttria, stabilized zirconia, ceria, stabilized zirconia, zirconia ceramics, alumina ceramics, oxonium or mixtures thereof.

6. A preassembled acetabular cup prosthesis according to any one of Claims 1 to 5 wherein an inner surface of the outer shell and an outer surface of the liner are configured so that there is a corresponding taper fit arrangement.

7. A preassembled acetabular cup prosthesis according to any one of Claims 1 to 6 wherein an adhesive is used to secure the liner in the shell.

8. A preassembled acetabular cup prosthesis according to Claim 7 wherein the adhesive is an anaerobic adhesive.
